(19) Europäisches Patentamt
European Patent Office
Office européen des brevets

(11) **EP 2 397 185 B1**

(12) **EUROPEAN PATENT SPECIFICATION**

(45) Date of publication and mention
of the grant of the patent:
**12.08.2015 Bulletin 2015/33**

(51) Int Cl.:
*A61B 5/00* *(2006.01)*          *A61B 5/02* *(2006.01)*
*A61B 5/11* *(2006.01)*          *A61B 5/0215* *(2006.01)*
*A61N 1/365* *(2006.01)*

(21) Application number: **10166535.4**

(22) Date of filing: **18.06.2010**

(54) **Blood pressure measurement with implantable medical device**

Blutdruckmessung mit implantierbarer medizinischer Vorrichtung

Mesure de la pression artérielle avec dispositif médical implantable

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(43) Date of publication of application:
**21.12.2011 Bulletin 2011/51**

(73) Proprietor: **St. Jude Medical AB**
**175 84 Järfälla (SE)**

(72) Inventors:
• **Björling, Anders**
**169 37 Solna (SE)**
• **Norén, Kjell**
**171 58 Solna (SE)**
• **Järverud, Karin**
**169 71 Solna (SE)**

(74) Representative: **Sjölander, Henrik et al**
**Aros Patent AB**
**P.O. Box 1544**
**751 45 Uppsala (SE)**

(56) References cited:
US-A- 4 566 456          US-A1- 2004 147 969
US-A1- 2009 299 211          US-B2- 6 915 162

**Description**

TECHNICAL FIELD

The embodiments relate to blood pressure measurements, and in particular to an implantable medical device capable of estimating blood pressure of a patient.

BACKGROUND

Blood pressure measurements are commonly used as diagnostic parameters in cardiac medicine. For example, mean arterial pressure (MAP) representing the average blood pressure in a patient and defined as the average arterial pressure during a single cardiac cycle is commonly used within the diagnostic field. Another common diagnostic parameter is pulse pressure (PP) defining the difference between systolic and diastolic blood pressure and reflects the change in blood pressure seen during a contraction of the heart.

Today blood pressure is measured with various equipment. For instance, arterial pressure is most commonly measured via a sphygmomanometer. Minimum systolic pressure value can be roughly estimated by palpation. The ausculatory method uses a stethoscope and a sphygmomanometer. This comprises an inflatable cuff placed around the upper arm attached to a mercury or aneroid manometer. Also ambulatory blood pressure devices are available on the marked for home monitoring.

However, for some patients it might be advantageous to monitor blood pressure continuously over certain period of times or conduct blood pressure measurements periodically or upon given events. This is in particular true for cardiac patients having an implantable medical device (IMD).

WO 02/28478 discloses a method to estimate the static intracardiac pressure from an intracardiac dynamic pressure sensor. The invention disclosed in this document is based on the finding that there is a relation between the sensed short-term dynamic pressure signal and the intravascular static pressure. A shortcoming with this sensor measuring technique is that the sensor has to be positioned at the relevant intravascular site in order to determine the intravascular static pressure at that site. For instance, pressures relating to the left ventricle must be conducted with the dynamic pressure sensor positioned inside the left ventricle.

US 5,129,394 and US 6,666,826 disclose a pressure sensor provided at the distal end of a left ventricular lead or provided on a sensing catheter introduced through the lumen of a left ventricular lead. An inflatable balloon or an occlusion device is provided proximal to the pressure sensor and is used to occlude the blood flow in a coronary vein in which the pressure sensor is provided. This blood flow occlusion is, according to the documents, a prerequisite in order to obtain an in vivo blood pressure that is proportional to the left ventricular pressure. If no such blood flow occlusion is conducted the result from the pressure measurement is random and not representative of left ventricular pressure.

US 6,915,162 discloses an implantable medical device having a pressure sensing arrangement to measure right ventricular pressure with a pressure sensor positioned in the right ventricle. The pressure sensor generates a pressure signal that is processed by a processor a diastolic pressure signal representing the ventricular pressure only during the diastolic phase of the heart cycle.

US 4,566,456 discloses a pacer with a pulse generator and a control circuitry. A pacer sensor is mounted on a pacing lead for sensing right ventricular systolic pressure. The measured right ventricular systolic pressure or a time derivative thereof is used to set the pacing rate of generating and applying pacing pulses from the pulse generator.

There is therefore still a need for conducting blood pressure measurements with an IMD that does not have the limitations or shortcomings of the prior art. In particular, it would be beneficial to conduct pressure measurements relating to the left ventricle but not requiring a positioning of the sensing equipment in the ventricle nor needing extra inflating or occluding devices.

SUMMARY

It is a general objective to provide an implantable medical device capable of conducting systemic blood pressure measurements.

It is a particular objective to achieve systemic blood pressure measurements having a sensor safely implanted at an implantation site commonly employed for cardiac leads connectable to an implantable medical device.

These and other objectives are met by embodiments as disclosed herein.

Briefly, an implantable medical device comprises a lead connecting arrangement configured to be connected to a cardiac lead to be implanted in or in connection with the ventricle of a subject heart. The cardiac lead comprises a cardiomechanic or cardiomechanical sensor configured to generate a deformation signal representative of the myocardial deformation. A derivative calculator of the implantable medical device receives the deformation signal and is configured to generate a deformation rate signal representative of the rate of myocardial deformation by calculating the derivative of the deformation signal with respect to time. A signal filter unit is configured to filter the deformation rate signal to improve signal quality and to get a filtered deformation rate signal. The filtered deformation rate signal is processed by a peak identifier that is configured to identify respective maximum deformation rate values in the filtered deformation rate signal for multiple cardiac cycles. A pressure calculator then calculates a value representative of the systemic blood pressure of the subject pressure calculator then calculates a value representative of the systemic blood pressure of the subject based on a combination of the respective maximum deformation rate values identified by the peak identifier.

An aspect of the embodiments relates to a method for estimating systemic blood pressure of a subject having an implantable medical device connected to a cardiac lead implanted in or in connection with a ventricle of the subject's heart. The cardiac lead comprises a cardiomechanic sensor configured to generate a deformation signal representative of the myocardial deformation. The method comprises generating a deformation rate signal representative of the rate of myocardial deformation by calculating the derivative of the deformation signal with respect to time. The deformation rate signal is filtered to get a filtered deformation rate signal. The method also comprises identifying respective maximum deformation rate values in the filtered deformation rate signal for multiple cardiac cycles. These respective maximum deformation rate values are then combined to get a calculated value representative of the systemic blood pressure of the subject.

The embodiments allow measuring and monitoring systemic blood pressure at any time for a subject having an implantable medical device. No dedicated visits to the physician are thereby needed to conduct blood pressure measurements. As a consequence, the implantable medical device can, in real-time, detect various malicious conditions to the subject and the heart, which can be seen as a significant change in systemic blood pressure. Additionally, a dynamic operation of the implantable medical device is possible based on the blood pressure measurements.

In contrast to the prior art, the embodiments allow reliable systemic blood pressure measurements without any need for any blood vessel obstructing equipment or the risk of implanting the pressure sensor in the left ventricle.

BRIEF DESCRIPTION OF THE DRAWINGS

The invention, together with further objects and advantages thereof, may best be understood by making reference to the following description taken together with the accompanying drawings, in which:

Fig. 1 is a schematic overview of a patient having an implantable medical device according to an embodiment and an external data processing unit capable of conducting wireless communication with the implantable medical device;

Fig. 2 is a schematic illustration of an implantable medical device according to an embodiment;

Figs. 3A and 3B illustrate different embodiments of cardiac lead sets that can be used in connection with an implantable medical device;

Fig. 4 illustrates a cross section of an embodiment of a cardiomechanic sensor;

Figs. 5A to 5D illustrate CMES signals for multiple cardiac cycles together with the IEGM for four pigs having the CMES sensor placed in a coronary left lateral vein;

Fig. 6 illustrates ECG, aortic pressure (AP), d(CMES)/dt from a CMES sensor located in a left sided coronary vein and average flow of the carotid artery recorded during a drug provocation for a time duration of 1500 s and each division is 30 s;

Fig. 7 is a zoom in of the signals presented in Fig. 6 during normal condition before the drug provocation;

Fig. 8 is a zoom in of the signals presented in Fig. 6 during the peak pressure response under the drug provocation;

Fig. 9 is a zoom in of the signals presented in Fig. 6 during a heart beat;

Fig. 10 shows the correlation between the blood pressure as determined according to an embodiment and the mean arterial pressure;

Fig. 11 shows the correlation between the blood pressure as determined according to an embodiment and the pulse pressure;

Fig. 12 is a flow diagram illustrating a method of determining blood pressure according to an embodiment;

Fig. 13 is a flow diagram illustrating an embodiment of the filtering step in Fig. 12; and

Fig. 14 is a flow diagram illustrating additional steps of the method in Fig. 12.

DETAILED DESCRIPTION

Throughout the drawings, the same reference numbers are used for similar or corresponding elements.
The embodiments generally relate to devices and methods capable of estimating systemic blood pressure in a subject, preferably mammalian subject and more preferably a human subject, by means of an implantable medical device (IMD) connectable to an implantable cardiomechanic sensor.
The embodiments are based on the finding that it is possible to use a cardiomechanic sensor configured to generate a deformation signal representative of the myocardial deformation of the cardiac myocardium in the subject's heart together with a special processing of the deformation signal to get a value representative of the systemic blood pressure in the subject.

A key advantage of the embodiments is that systemic blood pressure measurements are possible using a cardiomechanic sensor provided in a coronary vein of the heart. Hence, the embodiments enable systemic blood pressure measurements without the need of having the sensor provided in the left ventricle, which is generally not desired due to an increased risk of the formation of emboli.

The cardiomechanic sensor of the embodiments generates a deformation signal that is representative of the myocardial deformation. There is a relation between myocardial deformation and strain in terms of strain being equal to relative deformation. Hence, the signal from the cardiomechanic sensor could also be representative of the strain of the myocardium.

Without being bound by theory, it is known that the ambient or systemic blood pressure affects the cardiac output and stroke volume through Frank-Starling's law. An increase in contractility of the heart tends to increase stroke volume. Thus, an increased stroke volume is associated with an increased contractility. A positive value of the rate of myocardial deformation or the strain rate is a measure of compliance since the maximum (positive) deformation rate during a cardiac cycle represents the maximum speed at which the myocardium increases in size in the current cardiac cycle. Generally, this maximum increase in size occurs in early diastole or at the very end of systole. A positive deformation indicates an expansion of the myocardium and a negative deformation indicates a contraction. Thus, the speed at which the heart contracts, i.e. contractility, is manifested by a negative deformation rate. The speed at which the heart relaxes, i.e. compliance, is manifested by a positive deformation rate.

If the heart's size has decreased considerably during systole, i.e. has had a negative average deformation, then during diastole there must be a high average deformation. A high average deformation is not necessarily the same as a high peak deformation rate, although it is very likely that an increase in average deformation would also affect the peak deformation rate. In other words, there is a connection between high contractility, generating a high deformation in systole and a high peak deformation rate, indicating a high compliance.

Hence, there is a link between deformation rate as obtained by processing the deformation signal from the cardiomechanic sensor and ambient or systolic blood pressure.

The embodiments therefore use an implantable cardiomechanic sensor generating a deformation signal representative of the myocardial deformation and process the deformation signal to get a deformation rate signal from which a value representative of systemic blood pressure of the subject is obtainable according to the embodiments.

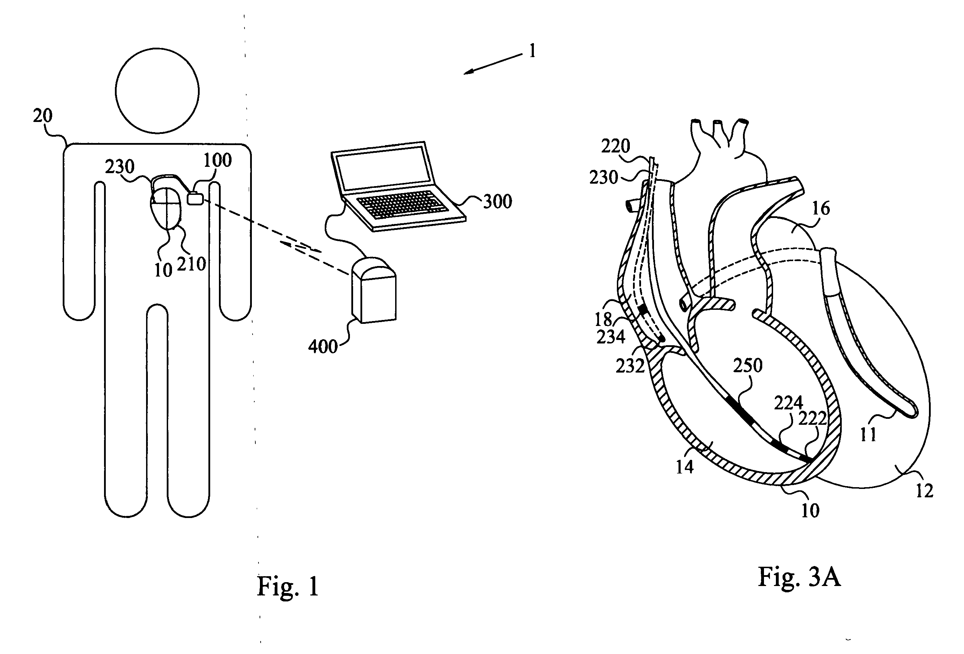

Fig. 1 is a schematic overview of a system 1 comprising an implantable medical device (IMD) 100 according to the embodiments and a non-implantable data processing device 300. In the figure, the IMD 100 is illustrated implanted in a human patient 20 and as a device that monitors and/or provides therapy to the heart 10 of the patient 20. The patient 20 must not necessarily be a human patient but can instead be an animal patient, in particular a mammalian patient, in which an IMD 100 can be implanted. The IMD 100 can be in the form of a pacemaker, cardiac defibrillator or cardioverter, such as implantable cardioverter-defibrillator (ICD). The IMD 100 is, in operation, connected to one or more, two in the figure, cardiac leads 210, 230 inserted into different heart chambers, the right atrium and left ventricle in the figure, or elsewhere provided in connection with a heart chamber.

According to the embodiments, the IMD 100 is connectable to at least one cardiomechanic sensor that is implanted in or in connection with a ventricle of the heart 10. The cardiomechanic sensor is advantageously arranged on a cardiac lead 210 configured to be connected to the IMD.

The figure also illustrates an external data processing device 300, such as programmer or clinician's workstation, that can communicate with the IMD 100, optionally through a communication device 400 that operates similar to a base station on behalf of the data processing device 300. As is well known in the art, such a data processing device 300 can be employed for transmitting IMD programming commands causing a reprogramming of different operation parameters and modes of the IMD 100. Furthermore, the IMD 100 can upload diagnostic data descriptive of different medical parameters or device operation parameters collected by the IMD 100. Such uploaded data may optionally be further processed in the data processing device 300 before display to a clinician. In the light of the present invention, the IMD 100 can transmit diagnostic data in terms of values representative of systemic blood pressure and/or other diagnostic data relating to pressure measurements. The data processing device 300 can further be used to transmit compensation factors that can be used by the IMD 100 to calculate absolute pressures, such as absolute mean arterial pressure (MAP) and/or pulse pressure (PP) from the value representative of systemic blood pressure as is further described herein.

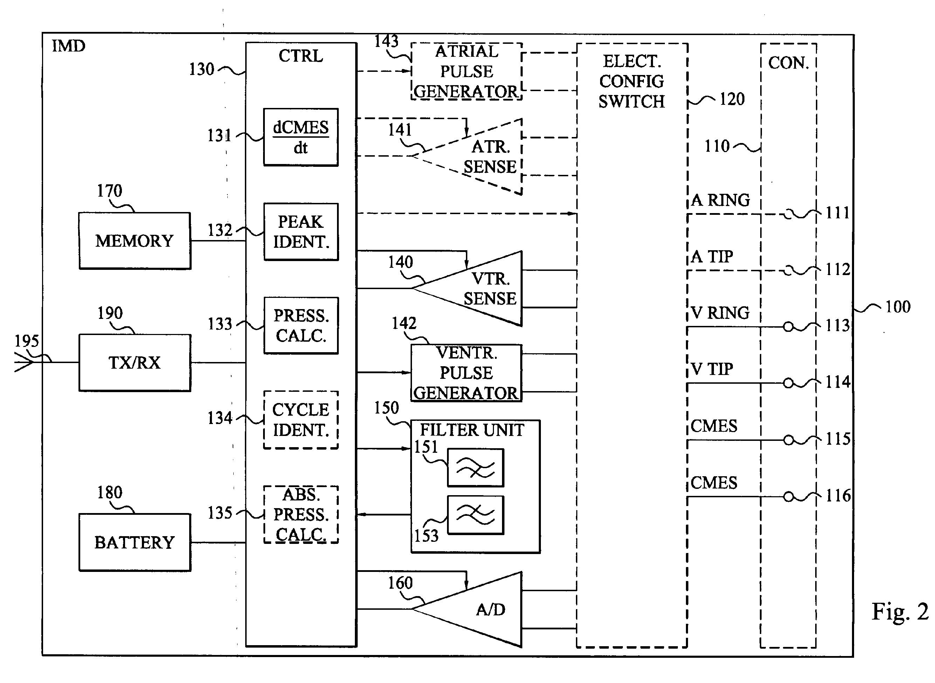

Fig. 2 illustrates an embodiment of an IMD 100 suitable for delivering cardiac therapy to a heart of a subject. The figure is a simplified block diagram depicting various components of the IMD 100. While a particular multi-chamber device is shown in the figure, it is to be appreciated and understood that this is done merely for illustrative purposes. Thus, the techniques and methods described below can be implemented in connection with other suitably configured IMDs. Accordingly, the person skilled in the art can readily duplicate, eliminate, or disable the appropriate circuitry in any desired combination to provide an IMD capable of treating the appropriate heart chamber(s) with pacing stimulation and also cardioversion and/or defibrillation.

The IMD 100 comprises a housing, often denoted as can or case in the art. The housing can act as return electrode for unipolar leads, which is well known in the art. The IMD 100 also comprises a lead connector or input/output (I/O) 110 having, in this embodiment, a plurality of terminals 111-116. These terminals 111-114 are configured to be connected to matching electrode terminals of one or more cardiac leads connectable to the IMD 100 and the lead connectors 110. In addition, the lead connector 110 comprises at least one and preferably two terminals 115, 116 arranged to be connected to matching terminals of a cardiac lead equipped with a cardiomechanic sensor. This at least one terminal 115, 116 therefore receives the deformation signal representative of the myocardial deformation generated by the cardiomechanic sensor, denoted cardiomechanic electric sensor (CMES) in the figure.

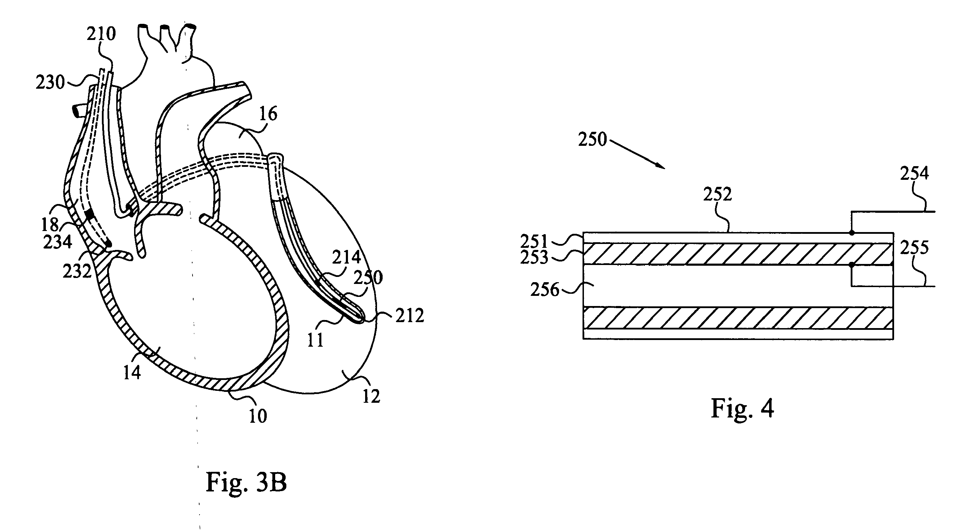

With reference to Figs. 2 and 3B, the lead connector 110 is configured to be, during operation in the subject body, electrically connectable to, in this particular example, a right atrial lead 230 and a left ventricular lead 210. The electrode connector 110 consequently comprises terminals 111, 112 that are electrically connected to matching electrode terminals of the atrial lead 230 when the atrial lead 230 is introduced in the lead connector 110. For instance, one of these terminals 112 can be designed to be connected to a right atrial tip terminal of the atrial lead 230, which in turn is electrically connected through a conductor running along the lead body to a tip electrode 232 present at the distal end of the atrial lead 230 in the right atrium 18 of the heart 10. A corresponding terminal 111 is then connected to a right atrial ring terminal of the atrial lead 230 that is electrically connected by another conductor in the lead body to a ring electrode 234 present in connection with the distal part of the atrial lead 230, though generally distanced somewhat towards the proximal lead end as compared to the tip electrode 232.

In an alternative implementation, the IMD 100 is not connectable to a right atrial lead 230 but instead to a left atrial lead configured for implantation in the left atrium 16. A further possibility is to have an IMD 100 with an electrode connector 110 having sufficient terminals to allow the IMD 100 to be electrically connectable to both a right atrial lead 230 and a left atrial lead. Though, it is generally preferred to have at least one electrically connectable atrial lead in order to enable atrial sensing and pacing, the IMD 100 does not necessarily have to be connectable to any atrial leads. In such a case, the terminals 111, 112 of the electrode connector 110 can be omitted.

In order to support left chamber sensing and pacing, the lead connector 110 further comprises a left ventricular tip terminal 114 and a left ventricular ring terminal 113, which are adapted for connection to a left ventricular tip electrode 212 and a left ventricular ring electrode 214 of the left ventricular lead 210 implantable in connection with the left ventricle 12, see Fig. 3B. A left ventricular lead 210 is typically implanted in the coronary venous system 11 for safety reasons although implantation inside the left ventricle 12 has been proposed in the art. In the following, "left ventricular lead" 210 is used to describe a cardiac lead designed to provide sensing and pacing functions to the left ventricle 12 regardless of its particular implantation site, i.e. inside the left ventricle 12 or in the coronary venous system 11.

The left ventricular lead 210 is equipped with a cardiomechanic sensor 250 according to the embodiments, such as a cardiomechanic sensor 250 of the CMES type. The cardiomechanic sensor 250 can be arranged anywhere along the portion of the left ventricular lead 210 that is in contact with and arranged in connection with the left ventricle 12. Hence, the cardiomechanic sensor 250 could be provided at the most distal end of the left ventricular lead 210, at a position upstream of the distal end, such as between the tip electrode 212 and the ring electrode 214 or upstream of the ring electrode 214. If the left ventricular lead 210 is of a so-called multi-electrode lead, such as quadropolar lead having four ring electrodes, the cardiomechanic sensor 250 could be provided in between two such electrodes. The CMES material can actually be placed onto one of the ring electrodes or tip electrode. In such a case, the CMES sensor 250 occupies the same or at least a portion of the same lead surface as the electrode. In a preferred embodiment, the electrode is then mainly employed for sensing and preferably not any pacing.

Fig. 3A illustrates an alternative embodiment, in which the cardiomechanic sensor 250 is arranged on a right ventricular lead 220 implanted in the right ventricle 14 of the heart 10. The right ventricular lead 220 comprises, in this embodiment, a tip electrode 222 and a ring electrode 224, which could be electrically connected to matching terminals of the lead connector 110 of the IMD 100. The discussion presented above regarding the position of the cardiomechanic sensor 250 on the left ventricular lead applies mutatis mutandis to the right ventricular lead 220.

In yet another embodiment, the IMD 100 is connected to both a left ventricular lead and a right ventricular lead and optionally further to at least one atrial lead. In such a case, any of or both the left and right ventricular lead can be equipped with a cardiomechanic sensor.

The cardiomechanic sensor must not necessarily be arranged on the same cardiac lead as the electrodes of the left or right ventricle. In clear contrast, a dedicated sensor lead or catheter can then be used that does not comprise any electrodes but merely the cardiomechanic sensor. Such a sensor lead can then generally be less complex as compared to constructing a cardiac lead having both pacing and/or sensing electrodes and a cardiomechanic sensor. However, using a dedicated sensor lead generally implies that the IMD has to be connected to a further lead and this further lead has to be transplanted into the heart in addition to the normal cardiac lead(s).

It is possible to use an IMD that is not connected to a single cardiomechanic sensor but rather multiple such cardiomechanic sensors. These multiple sensors can all be provided on the same lead or on different leads in or connection with the same ventricle or distributed among both ventricles. In such a case, the further processing of the deformation signals as disclosed herein can then be performed for each of the deformation signals. The final value representative of the systemic blood pressure is preferably an average of respective such values obtained from each of the cardiomechanic sensors. Alternatively, averaging can be performed anywhere in the processing up to the final pressure value.

The cardiomechanic sensor preferably comprises a piezoelectric material. Piezoelectric materials generate a charge when subject to mechanical stress or strain, with the magnitude of charge dependent upon the magnitude of the stress or strain. A sensor comprised of such piezoelectric material can be arranged for detection of myocardial deformation and generate raw signals of myocardial deformation.

A cardiomechanic sensor preferably comprises one or more piezoelectric transducers, which convert mechanical motion into electric signals. Such a cardiomechanic sensor 250 of CMES type is illustrated in cross section in Fig. 4. The cardiomechanic sensor 250 comprises, in this particular embodiment, a tubular or annular piezoelectric element 251 either self-supporting or disposed on a supporting structure. In a particular embodiment, conductors 252, 253 contact the inner and outer surfaces of the piezoelectric element 251. An electrical connection 254 is then coupled to the outer conductor 252 and another electrical connection 255 is connected to the inner conductor 253. This inner conductor 253 can optionally constitute the supporting structure fro the piezoelectric element 251. These electrical connections 254, 255 preferably run along the length of the lead body up to terminals arranged in connection with the proximal end of the lead and connectable to the terminals 115, 116 in Fig. 2.

The cardiomechanic sensor 250 is preferably designed and dimensioned to be arranged on a cardiac lead and in particular a ventricular lead. In such a case, the outer diameter of the cardiomechanic sensor 250 can be similar to the outer diameter of the cardiac lead. In some embodiments, one or more electrodes of the cardiac lead can be disposed over at least a portion of the cardiomechanic sensor 250.

The cardiomechanic sensor 250 preferably comprises a longitudinal passageway or bore 256 to permit routing of electrical connections therethrough.

The conductor 252 and optionally conductor 253 comprise any suitable biocompatible, electrically conducting material known in the art, for example, titanium, including titanium, platinum, carbon, niobium, tantalum, gold, combinations thereof including alloys of the metallic materials presented above, and titanium nitride.

An elastomer can be disposed over the cardiomechanic sensor 250. The elastomer can then be selected from biocompatible elastomers that are suitable for implantation in the animal body, such as silicones, polyurethanes, ethylene-propylene copolymers, fluorinated elastomers and combinations thereof.

The piezoelectric material 251 of the cardiomechanic sensor 250 can be of a relatively hard material, thereby permitting reliable measurements with only small deflections of the piezoelectric material 251. Preferred such piezoelectric materials include ceramic ferroelectric particles, lead zirconate titanate (PCT), barium titanate, sodium potassium niobate. A non-limiting example of piezoelectric material that can be used is described in U.S. Patent No. 6,526,984 and has the general formula of $Na_{0.5}K_{0.5}NbO_3$.

Instead of using a piezoelectric material the cardiomechanic sensor can use a conductive polymer that has resistance that changes as a function of deformation. By measuring the resistance of the conductive polymer, the cardiomechanic deformation can be determined. Non-limiting examples of conductive polymers include polyacetylene, polyaniline, polypyrrole.

More information of the design of the cardiomechanic sensor can be found in U.S. Patent Application No. 2009/0312814.

With reference to Fig. 2, the housing can act as return electrode as previously mentioned above. In such a case, the lead connector 110 can have a dedicated terminal (not illustrated) connected to the housing electrode.

The IMD 100 as illustrated in Fig. 2 comprises an optional atrial pulse generator 143 and a ventricular pulse generator 140 that generate pacing pulses for delivery by the atrial lead(s) and the ventricular lead(s) preferably through an electrode configuration switch 120.

It is understood that in order to provide stimulation therapy in different heart chambers, the atrial and ventricular pulse generators 140, 143 may include dedicated, independent pulse generators, multiplexed pulse generators, or shared pulse generators. The pulse generators 140, 143 are controlled by a controller 130 via appropriate control signals, respectively, to trigger or inhibit the stimulating pulses.

The IMD 100 also comprises the controller 130, preferably in the form of a programmable microcontroller 130 that controls the operation of the IMD 100. The controller 130 typically includes a microprocessor, or equivalent control circuitry, designed specifically for controlling the delivery of pacing therapy, and may further include RAM or ROM memory, logic and timing circuitry, state machine circuitry, and I/O circuitry. Typically, the controller 130 is configured to process or monitor input signal as controlled by a program code stored in a designated memory block. The type of controller 130 is not controller 130 is not critical to the described implementations. In clear contrast, any suitable controller may be used that carries out the functions described herein. The use of microprocessor-based control circuits for performing timing and data analysis functions are well known in the art.

The controller 130 further controls the timing of the stimulating pulses, such as pacing rate, atrioventricular interval (AVI), atrial escape interval (AEI) etc. as well as to keep track of the timing of refractory periods, blanking periods, noise detection windows, evoked response windows, alert intervals, marker channel timing, etc.

A preferred electronic configuration switch 120 includes a plurality of switches for connecting the desired terminals 111-114 to the appropriate I/O circuits, thereby providing complete electrode programmability. Accordingly, the electronic configuration switch 120, in response to a control signal from the controller 130, determines the polarity of the stimulating pulses (e.g., unipolar, bipolar, combipolar, etc.) by selectively closing the appropriate combination of switches (not shown) as is known in the art.

An optional atrial sensing circuit or detector 141 and a ventricular sensing circuit or detector 142 are also selectively coupled to the atrial lead(s) and the ventricular lead(s) through the switch 120 for detecting the presence of cardiac activity in the heart chambers. Accordingly, the atrial and ventricular sensing circuits 141, 142 may include dedicated sense amplifiers, multiplexed amplifiers, or shared amplifiers. The switch 120 determines the "sensing polarity" of the cardiac signal by selectively closing the appropriate switches, as is also known in the art. In this way, the clinician may program the sensing polarity independent of the stimulation polarity. The sensing circuits are optionally capable of obtaining information indicative of tissue capture.

Each sensing circuit 141, 142 preferably employs one or more low power, precision amplifiers with programmable gain and/or automatic gain control, bandpass filtering, and a threshold detection circuit, as known in the art, to selectively sense the cardiac signal of interest.

The outputs of the atrial and ventricular sensing circuits 141, 142 are connected to the controller 130, which, in turn, is able to trigger or inhibit the atrial and ventricular pulse generators 140, 143, respectively, in a demand fashion in response to the absence or presence of cardiac activity in the appropriate chambers of the heart.

Furthermore, the controller 130 is also capable of analyzing information output from the sensing circuits 141, 142 and/or a signal sensing unit or data acquisition unit 160 to determine or detect whether and to what degree tissue capture has occurred and to program a pulse, or pulse sequence, in response to such determinations. The sensing circuits 141, 142, in turn, receive control signals over signal lines from the controller 130 for purposes of controlling the gain, threshold, polarization charge removal circuitry, and the timing of any blocking circuitry coupled to the inputs of the sensing circuits 141, 142 as is known in the art.

According to the embodiments cardiac signals are applied to inputs of the data acquisition unit 160 connected to the electrode connector 110. The data acquisition unit 160 is preferably in the form of an analog-to-digital (A/D) data acquisition unit 160 configured to acquire intracardiac electrogram (IEGM) signals, convert the raw analog data into a digital signal, and store the digital signals for later processing and/or transmission to the programmer by a transceiver 190. The data acquisition unit 160 is coupled to the atrial lead and/or the ventricular lead through the switch 120 to sample cardiac signals across any pair of desired electrodes.

The IMD 100 comprises a derivative calculator 131 configured to process the deformation signal obtained from the cardiomechanic sensor through the terminals 115, 116 and the preferred switch 120. The derivative calculator 131 in particular generates a deformation rate signal representative of the rate of the myocardial deformation. Thus, the derivative calculator 131 calculates the derivative with respect to time of the myocardial deformation as represented by the deformation signal. The deformation rate signal will, thus, comprise multiple samples, where each sample has a data element corresponding to the deformation rate at the point in time represented by the particular sample. The derivative calculator 131 can, in a simple embodiment, generate the deformation rate signal by calculating the difference in data elements between successive samples in the deformation signal, i.e. $dfr_i = dS_i - dS_{i-1}$, where $dfr_i$ represents sample / in the deformation rate signal and $ds_{i/i-1}$ represents sample $i/i-1$ in the deformation signal.

A signal filter unit 150 is arranged in the IMD 100 for filtering the deformation rate signal from the derivative calculator 131. The filter unit 150 is in particular arranged in order to suppress noise and generally improve the quality of the deformation rate signal. The output from the filter unit 150 is a filtered deformation rate signal.

A peak identifier 132 of the IMD 100 is configured to identify respective maximum deformation rate values in the filtered deformation rate signal for multiple cardiac cycles. In a particular embodiment, the peak identifier 132 simply parses through the samples of the filtered deformation rate signal corresponding to a cardiac cycle in order to find the largest value, which corresponds to the maximum rate value for that cardiac cycle. The peak identifier 132 must not necessarily parse through all the samples corresponding to a cardiac cycle in order to identify the maximum deformation rate value. As has previously been discussed, this maximum in deformation rate generally occurs in early diastole or at the very end of systole. Hence, it is generally sufficient if the peak identifier 132 merely parses through the samples corresponding to the cardiac cycle coinciding with end of systole-early diastole, generally occurring around the T-wave as detectable in the IEGM signal.

The peak identifier 132 preferably identifies the maximum deformation rate value in each out of multiple cardiac cycles. These multiple cardiac cycles are preferably consecutive cardiac cycles. However, skipping one or a few cardiac cycles generally does not have any large impact on the processing so it is not a prerequisite that the multiple cardiac cycles are successive cardiac cycles.

When conducting pressure measurements, the IMD 100 generally collects the deformation signal from the cardiomechanic sensor for some defined period of time. In such a case, the peak identifier 132 can advantageously identify a respective maximum deformation rate value in the filtered deformation rate signal for each full cardiac cycle encompassed by the period of time to get as many maximum deformation rate values as possible.

The IMD 100 also comprises a pressure calculator 133 that is configured to calculate a value representative of the systemic blood pressure of the IMD patient based on a combination of multiple maximum deformation rate values identified by the peak identifier 132. Thus, the pressure calculator 133 co-processes multiple, i.e. at least two, maximum deformation rate values in order to get a blood pressure presenting value. These multiple maximum deformation rate values are preferably identified by the peak identifier from consecutive cardiac cycles. The generated blood pressure representing value is of high diagnostic value and is therefore advantageously stored in a memory 170 of the IMD 100 for later uploading to the data processing unit by means of transmitter 190 or transceiver having connected antenna 195. The blood pressure representing value can also be used for diagnostic purposes by the IMD 100 itself and control the operation of the IMD 100, which is further discussed herein.

The filter unit 150 of the IMD 100 preferably comprises a low pass filter 153 configured to low pass filter the deformation rate signal to remove or at least suppress noise from the deformation rate signal. Experiments have been successfully conducted with a 4th order low pass 30 Hz filter.

The filter unit 150 can alternatively or preferably in addition comprise a high pass filter 151 that is configured to high pass filter the deformation rate signal in order to remove or at least suppress any DC component from the deformation rate signal. Experiments have been successfully conducted with a 2nd order high pass 0.1 Hz filter.

Thus, the filter unit 150 preferably both conduct high pass and low pass filtering to remove or suppress the DC component and noise from the deformation rate signal and further to improve the signal quality.

Both the low pass and high pass filters can be either analog or digital. In case of analog filtering, the filter can be switch cap-based or component-based using resistors, capacitors and operational amplifiers. In case of the digital domain, the filter can either have a finite or infinite impulse response, i.e. be either so-called FIR or IIR filters. Typical IIR filters can be Butterworth, Chebyshev, elliptical, Bessel or other.

The IMD 100 preferably comprises a cardiac cycle identifier 134 configured to identify a start point and an end point of a cardiac cycle in the filtered deformation rate signal. The cardiac cycle identifier 134 advantageously identifies the start sample corresponding to the start point of the cardiac cycle and the end sample corresponding to the end point of the cardiac cycle in the filtered deformation rate signal. The cardiac cycle identifier 134 preferably identifies the start and end samples based on the signal representative of electric activity of at least a portion of the heart generated by the data acquisition unit 160. It is well known in the art that the start and end points of a cardiac cycle is relatively easily identifiable from an IEGM signal generated by the data acquisition unit 160. The cycle identifier 134 can then simply identify the samples in the filtered deformation rate signal that coincide in time with the start and end points as identified in the IEGM signal. The mapping of the start and end points in the IEGM signal to the start and end sampled in the filtered deformation rate signal is easily performed based on the relation in sampling frequency of the IEGM signal versus the deformation signal.

Once the cycle identifier 134 has identified the start sample and the end sample of a cardiac cycle, the peak identifier 132 can parse through the samples in the filtered deformation rate signal between the start and end samples in order to find the maximum deformation rate for that cardiac cycle.

The cycle identifier 134 preferably identifies respective start and end sample for multiple, preferably consecutive cardiac cycles, in the filtered deformation rate signal. The peak identifier 132 can then identify respective maximum deformation rate values for each of these consecutive cardiac cycles or at least a portion thereof.

The pressure calculator 133 of the IMD 100 preferably calculates the value representative of the systemic blood pressure based on the amplitudes or magnitudes of multiple maximum deformation rate values. Various processings are possible and within the scope of the embodiments. The pressure calculator 133 preferably calculates at least one value representative of the systemic blood pressure as the average of multiple maximum deformation rate values, preferably multiple consecutive maximum deformation rate values. Consecutive maximum deformation rate values correspond to respective maximum deformation rate values for consecutive cardiac cycles. In a particular embodiment, the pressure calculator 133 is configured to calculate a signal representative of the systemic blood pressure. This signal is obtained from a moving average of multiple consecutive deformation rate values: $ps_i \frac{1}{N} \sum_{j=i-(N-1)}^{i} fdrs_j$ , where $ps_i$

represents sample $i$ of the signal, $fdrs_j$ represents sample $j$ of the filtered deformation rate signal and N is a predefined positive number equal to or larger than two.

Averaging over multiple maximum deformation rate values is generally preferred since it decreases the effects of confounding factors that may impact the deformation signal from the cardiomechanic sensor. These effects can, for instance, be micro movements of the cardiomechanic sensor, beat-to-beat variability in the deformation rate, noise on the sensor, etc. By averaging over a period of time, corresponding to a number of cardiac cycles, these effects be canceled out or at least decrease in magnitude.

Instead of using a simple moving average, the pressure calculator 133 can use a weighted average with different weights for different maximum deformation rate values. In such a case, weights for more recent samples could be set higher as compared to maximum deformation rate values occurring further back in time.

Experimental studies have been successfully conducted with a moving average over 30 cardiac cycles. The number of heart cycles over which the deformation signal may be averaged is preferably in the range of 10 to 180 cycles.

It is also possible, most preferably in case of a continuous deformation measurement, to process the obtained maximum deformation rate values with an exponential averaging method or a so-called IIR filter. An exponentially weighted averaging method is a type of IIR filter well known in the art and it is preferable for implementation into a medical device as it does not require storing of a large set of filter coefficients and requires less calculations.

Although less preferred than using an average of maximum deformation rate values, the pressure calculator 133 could use the median of multiple maximum deformation rate value as the value representative of the systemic blood pressure of the patient.

The value or signal generated by the pressure calculator 133 is representative of the systemic blood pressure of the patient and is, in fact, a relative systemic blood pressure. In a particular embodiment, the IMD 100 can be configured to not only be capable of measuring relative systemic blood pressure but actually generate a value or signal representative of absolute systemic blood pressure, such as MAP and/or PP.

In such a case, the IMD 100 is employed together with a device capable of recording absolute blood pressure, such as MAP and PP. For instance, MAP and PP can be measured using a standard arm cuff blood pressure apparatus. Alternatively, any other well-known device for measuring MAP and/or PP, such as any of the devices mentioned in the background section, can be used. The values representative of systemic blood pressure from the pressure calculator 133 are then transmitted by the transmitter/transceiver 190 to the data processing unit 300 illustrated in Fig. 1 and optionally through the communication device 400. The data processing device 300 is further connected to the MAP and/or PP measuring device (not illustrated). Alternatively, the MAP and/or PP measurement results from this non-implanted device can be entered by the physician or some other person using the keyboards or other user input of the data processing device.

The data processing device 300 then calculates scale or mapping factors based on the relative systemic blood pressure values and the MAP and/or PP values. Two such factors are needed per blood pressure parameter so the pressure measurements conducted by the IMD 100 and the external MAP and/or PP measuring device have to be conducted for at least two different MAPs and/or PPs. This can, for instance, be achieved by letting the patient 20 walk on a treadmill or pedal on a bicycle. The more different MAPs and/or PPs that are tested the better the quality of the mapping factors. The data data processing unit 300 can use different well-known optimization techniques in order to derive the mapping factors that allow conversion or mapping of the value representative of relative systemic blood pressure from the pressure calculator into an absolute MAP or an absolute PP value. A non-limiting example of such an optimization technique is the method of least squares and variants thereof. Such a method can then be used to derive the factors of linear polynomial: $y=kx+m$, where y represents the absolute MAP or PP value, x is the value representative of system blood pressure from the pressure calculator 133 and k and m are the mapping factors determined by the data processing device 300.

The determined mapping factors can then be downloaded into the IMD 100 using the communication device. The mapping factors are received by the receiver/transceiver 190 and stored by the controller 130 in the memory 170. The memory 170 can therefore store one set of mapping factors for conversion into absolute MAP values, one set of mapping factors for conversion into absolute PP values or two sets of mapping factors, where one is used for absolute MAP conversion and the other for absolute PP conversion.

The IMD 100 preferably comprises an absolute pressure calculator 135 configured to calculate an absolute MAP value ($y_{MAP}$) representative of an absolute MAP of the patient and/or absolute PP value ($y_{PP}$) representative of an absolute PP of the patient based on the value calculated by the pressure calculator 133 and a MAP scaling factor ($k_{MAP}$) and a MAP offset factor ($m_{MAP}$) or a PP scaling factor (kpp) and a PP offset factor (mpp): $y_{MAP}=k_{MAP}x+m_{MAP}$ and $y_{PP}=k_{PP}x+m_{PP}$.

The controller 130 is further coupled to the memory 170 by a suitable data/address bus, wherein the programmable operating parameters used by the controller 130 are stored and modified, as required, in order to customize the operation of the IMD 100 to suit the needs of a particular patient. Such operating parameters define, for example, time threshold, pacing pulse amplitude, pulse duration, electrode polarity, rate, sensitivity, automatic features, time interval between pacing pulse of an applied pacing pulse sequence and the previously mentioned mapping factors.

The memory 170 may also advantageously store diagnostic data collected by the IMD 100. The diagnostic data include the IEGM signal from the data acquisition unit 160, the signal from the pressure calculator 133 and optionally absolute MAP and/or PP values or signal from the absolute pressure calculator 135.

Advantageously, the operating parameters of the IMD 100 may be non-invasively programmed into the memory 170 through the transceiver 190 in communication via a communication link with the previously described communication unit of the programmer. The controller 130 activates the transceiver 190 with a control signal. The transceiver 190 can alternatively be implemented as a dedicated receiver and a dedicated transmitter connected to separate antennas or a common antenna, preferably a radio frequency (RF) antenna 195.

The IMD 100 additionally includes a battery 180 that provides operating power to all of the circuits shown in Fig. 2.

In the figure the derivative calculator 131, the peak identifier 132, the pressure calculator 133 and the optional cardiac cycle identifier 134 and absolute pressure calculator 135 have been exemplified as being run by the controller 130.

These units can then be implemented as a computer program product stored on the memory 170 and loaded and run on a general purpose or specially adapted computer, processor or microprocessor, represented by the controller 130 in the figure. The software includes computer program code elements or software code portions effectuating the operation of the derivative calculator 131, the peak identifier 132, the pressure calculator 133, the cardiac cycle identifier 134 and absolute pressure calculator 135. The program may be stored in whole or part, on or in one or more suitable computer readable media or data storage means that can be provided in an IMD 100.

In an alternative embodiment, the derivative calculator 131, the peak identifier 132, the pressure calculator 133, the cardiac cycle identifier 134 and absolute pressure calculator 135 are implemented as hardware units either forming part of the controller 130 or provided elsewhere in the IMD 100.

The signal filter unit 150 has been illustrated as separate unit of the IMD 100. In an alternative embodiment, the filter unit 150 is implemented as forming part of the controller 130 in similarity to the other units implemented and run therein.

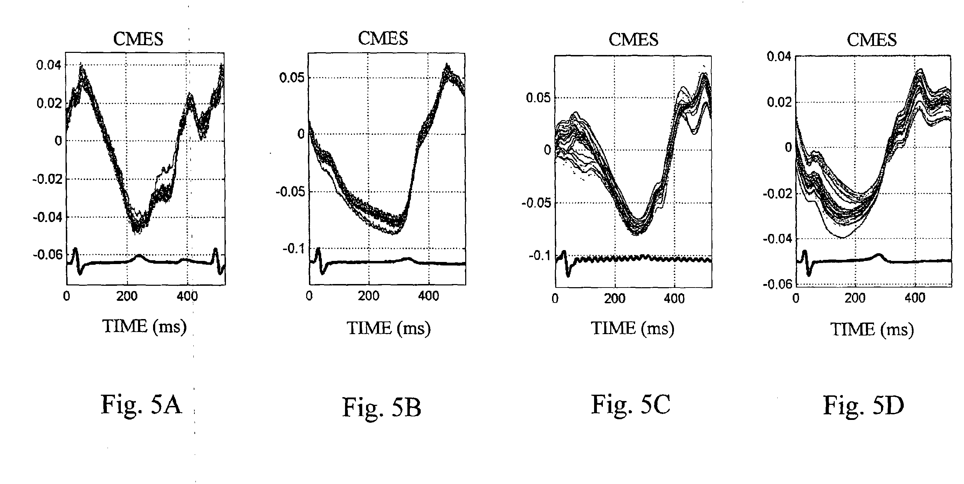

Figs. 5A-5D show the deformation signal from the cardiomechanic sensor for a number of cardiac cycles together with the IEGM for four different pigs. The cardiomechanic sensor was placed in a coronary left lateral vein and registers the deformation of the myocardium. It can be noted that all animals has a signal minimum at around the T wave, i.e. at early diastole/end of systole. At this time the ventricle has a small size, which corroborates the physiological interpretation of the deformation signal. The steepest positive derivative, i.e. the peak deformation rate, occurs approximately at the T wave of where systole goes into diastole for all animals.

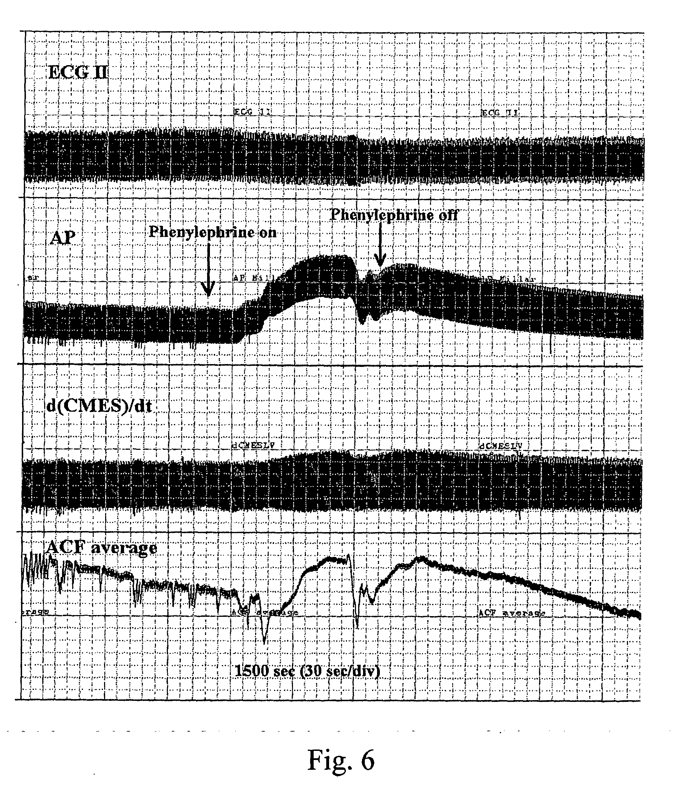

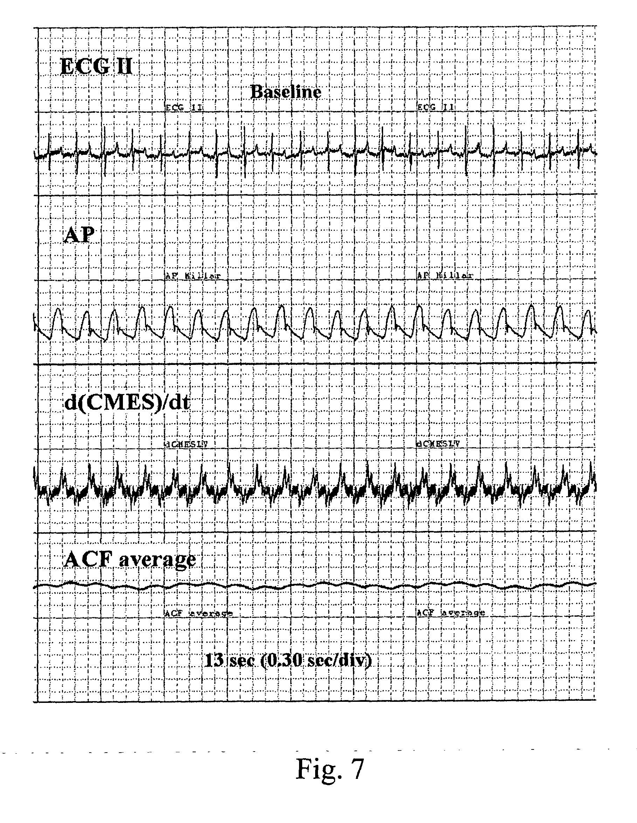

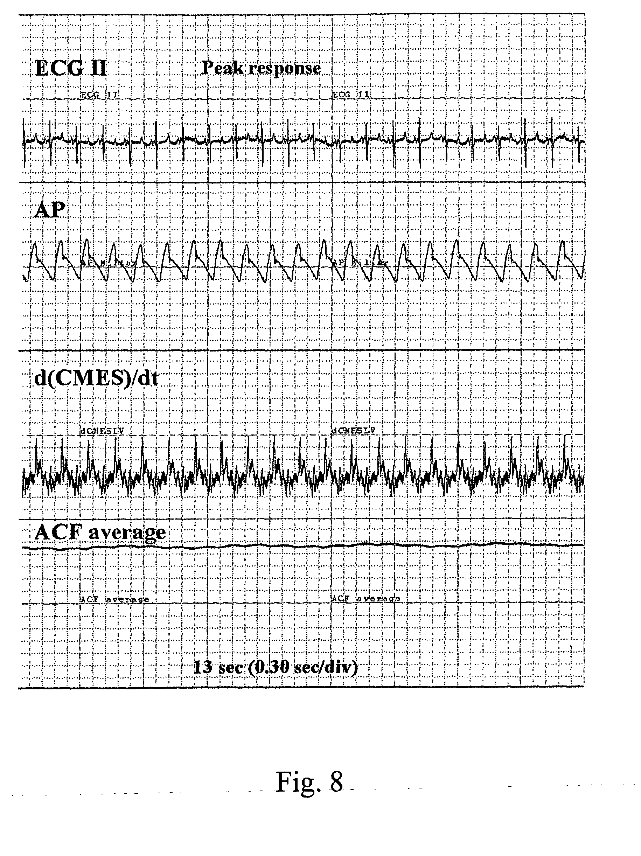

Fig. 6 illustrates signals recorded during a drug provocation with phenylephrine, which is an agent used to increase blood pressure. The time duration is 1500 s and each division is 30 s so the response is shown at high compression. At the top is the surface ECG followed by the aortic pressure recorded by a high fidelity pressure sensor. Next is the deformation rate signal prior filtering (d(CMES)/dt) obtained using a CMES sensor located in a left side coronary and sensing activities from the left ventricle. The bottom panel shows the average flow of the carotid artery. Fig. 7 is a zoom in of the signals in Fig. 6 during normal condition before the drug provocation. The peaks or maxima in the deformation rate signal can be observed during each heart cycle. Fig. 8 is a zoom in of the signals in Fig. 6 during the peak pressure response under the drug provocation. The peaks in the deformation rate signal have increased in amplitude as compared to Fig. 7.

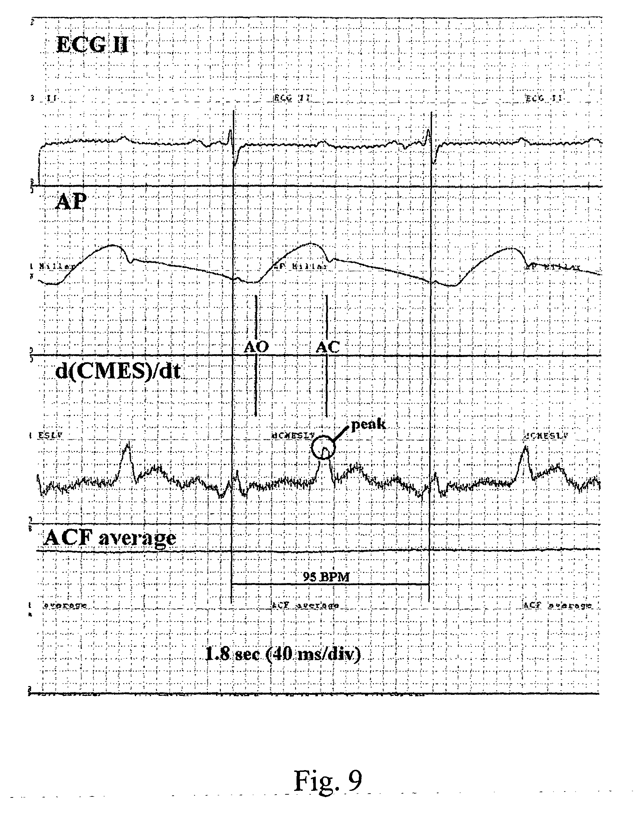

Fig. 9 shows in detail the peak or maximum in the deformation rate signal during a heart cycle. The opening (AO) and closure (AC) of the aortic valve are also marked in the figure. It is clear from the figure that the peak is coincident with the closure of the aortic valve.

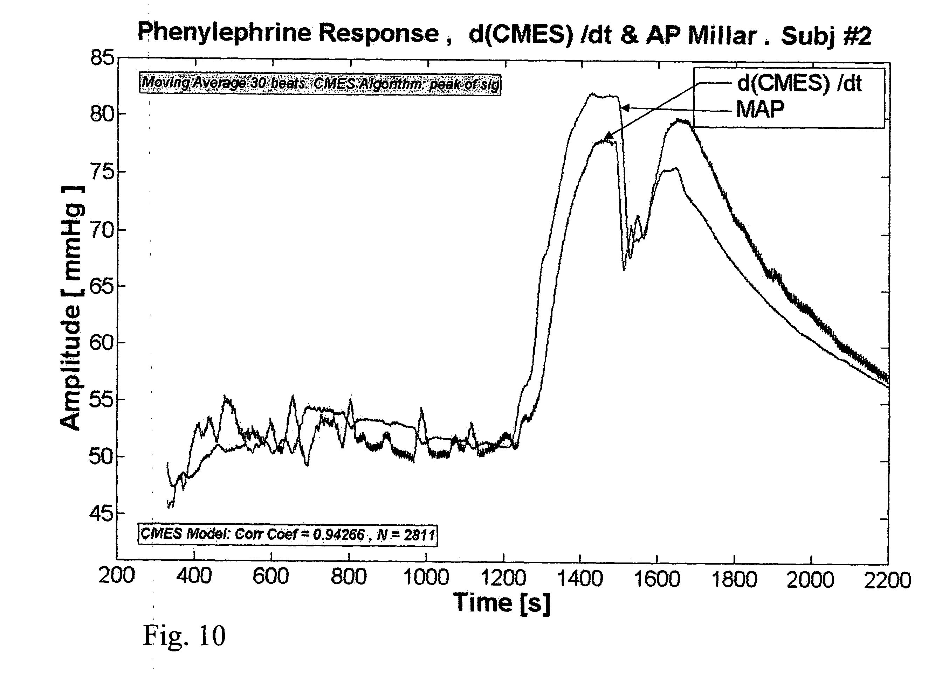

Fig. 10 shows the result of an embodiment when correlated to the mean arterial pressure. The signal representing systemic blood pressure (d(CMES)/dt) is obtained using a moving average over 30 cardiac cycles. The correlation coefficient between the signal and the MAP signal has a high value of 0.94.

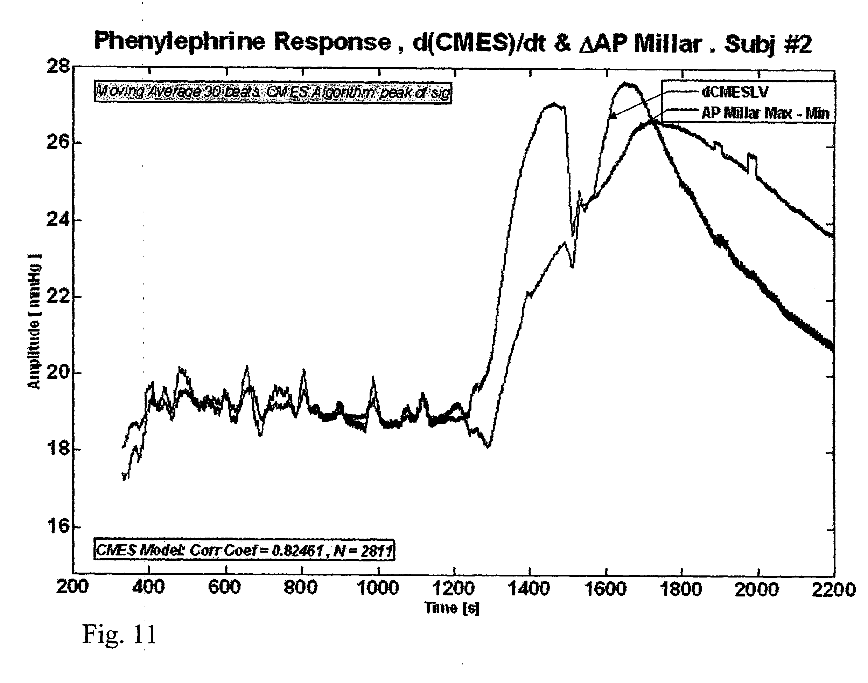

Fig. 11 shows the result of an embodiment when correlated to the pulse pressure. The signal representing systemic blood pressure (d(CMES)/dt) is obtained using a moving average over 30 cardiac cycles. The correlation coefficient between the signal and the PP signal has a high value of 0.82.

The above presented figures thereby confirm that the embodiments can be used to accurately generate a value or signal representative of systemic blood pressure in animals and furthermore can be used to obtain accurate representations of absolute MAP and/or PP values.

The embodiments were initially designed to obtain the systemic blood pressure value or signal using a cardiomechanic sensor implanted in connection with the left ventricle and advantageously in a coronary vein of the left ventricle. At this position the cardiomechanic sensor efficiently captures deformation of the myocardium of the left ventricle as the sensor is positioned close to or even in direct contact with the myocardium.

Experiments have also been conducted with the cardiomechanic sensor arranged in the right ventricle. It was then very surprising that the same cardiomechanic sensor as used in the left ventricle and the same signal processing could be employed and still get a very accurate value representative of the systemic blood pressure also with a right ventricular cardiomechanic sensor. It is speculated that this sensor will also capture the deformation of the myocardium partly from the myocardium of the right ventricle but also from myocardial deformations that are transplanted through the blood present in the right ventricle to thereby be captured by the cardiomechanic sensor.

Thus, although arranging the cardiomechanic sensor in connection with the left ventricle is thought to be preferred, the embodiments also cover implantation in or in connection with the right ventricle. Not all patients receive a lead in a left-sided coronary vein, why placing the sensor in the right ventricle may be the only option unless an extra lead is to be implanted.

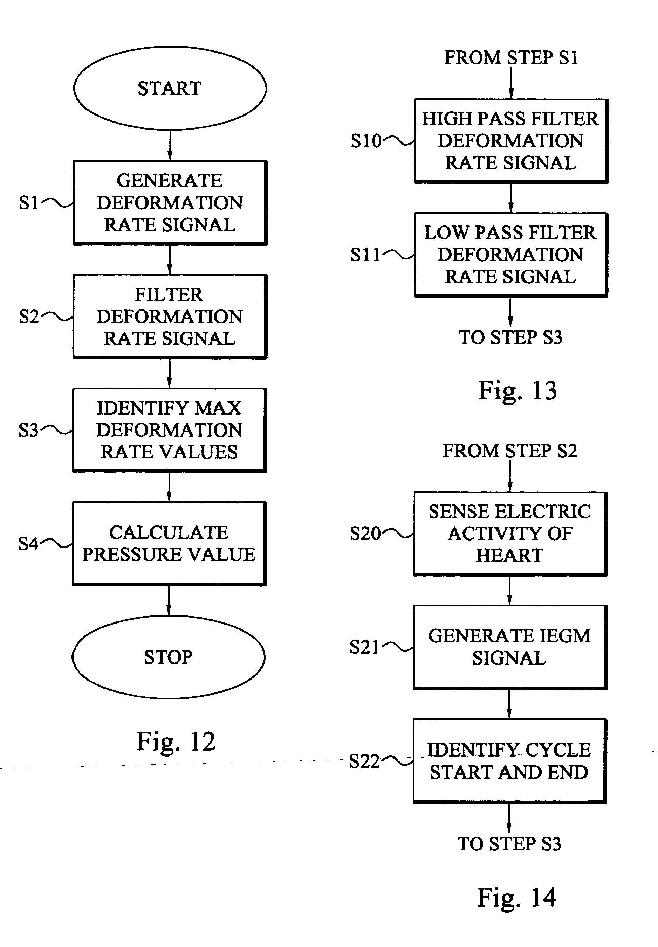

Fig. 12 is a flow diagram illustrating a method of estimating systemic blood pressure of a patient having an IMD connected to a cardiac lead implanted in or in connection with a ventricle of the heart and comprising a cardiomechanic sensor configured to generate a deformation signal representative of the myocardial deformation. The method starts in step S1 where a deformation rate signal representative of the rate of myocardial deformation is generated by calculating the derivative of the deformation signal with respect to time. The deformation rate signal is then filtered in step S2 to improve the quality thereof and obtain a filtered deformation rate signal. A next step S3 identifies maximum deformation rate values in multiple cardiac cycles in the filtered deformation rate signal. These multiple maximum deformation rate values or at least a portion thereof are used in step S4 to calculate a pressure value representative of system blood pressure for the patient.

The pressure value is preferably calculated as an average of the magnitudes or amplitudes of respective maximum deformation rate values for multiple cardiac cycles, preferably multiple consecutive cardiac cycles. Step S4 preferably generating not a single pressure value but rather a signal comprising multiple samples, each having a data element corresponding to the systemic blood pressure at the point in time associated with the particular sample.

An optional additional step of the estimating method involves calculating an absolute value representative of an absolute systemic blood pressure, such as MAP and/or PP, based on the pressure value and a scaling factor and offset factor as previously described.

Fig. 13 is a flow diagram illustrating a particular embodiment of the filtering step S2 in Fig. 12. The method continues from step S1 in Fig. 12. A next step S10 high pass filter the deformation rate signal to remove or suppress any DC component. A next step S11 low pass filters the deformation rate signal to remove or suppress noise. The method then continues to step S3 of Fig. 12. In an alternative embodiment, the order of the two filtering steps is switched, i.e. low pass filtering prior high pass filtering. Alternatively, one of the filtering steps can be omitted and in such a case preferably step S10.

Fig. 14 is a flow diagram illustrating additional, optional steps of the estimating method. The method continues from step S2 of Fig. 12. A next step S20 senses electric activity of least a portion of the heart. A signal, such as IEGM signal, representative of the electric activity is generated in step S21 and employed to identify the start and end points of cardiac cycles in the filtered deformation rate signal. A cardiac cycle can, for instance be defined as R-R interval, P-P interval or T-T interval or based on some other characteristic feature that is easily identified in the IEGM signal. The method then continues to step S3 of Fig. 12, where the maximum deformation rate value is determined from the identified start point to the identified end point in the filtered deformation rate signal.

The pressure value or signal generated according to the embodiments is not only of high diagnostic value itself. It can further be used by the IMD as is briefly discussed below.

One of the most common comorbidities of pacemaker and ICD patients is hypertension. By regularly measuring the absolute MAP or PP, the IMD acquires an objective trend of the hyper (or indeed hypo) tension of the patient. This trend can be viewed by the physician, e.g. at follow-ups. By studying the long term trend of the pressure, the effects of antihypertensive (or antihypotensive) drugs can be studied. Thus, the embodiments can aid in drug titration.

For ICD patients inappropriate shocks are a difficult problem. Fast supraventricular tachycardias (SVTs) are often misinterpreted as ventricular tachycardias (VTs) and ventricular therapy, such as shock or anti-tachycardia pacing (ATP) is applied. By studying the MAP and/or PP during the arrhythmia the IMD can conclude whether the arrhythmia is a VT or SVT. VT generally leads to a reduction in or low MAP and/or PP and therefore such a low MAP and/or PP can be used to select a more aggressive anti-arrhythmia treatment by the IMD, such as to shock the patient, than if the MAP and/or PP is high during the arrhythmia event. If the MAP and/or PP is high the probability is high that the patient is conscious and then it is better to try a less painful type of therapy, such as ATP, or to withhold therapy altogether.

Vasovagal syncope has two main forms: a cardioinhibitory and a vasodilatory type. In the first case, the heart rate of the patient is decreased and the blood pressure is thereby decreased. This can today be detected by an IMD without any cardiomechanic sensor and using heart rate monitoring. In the second case, the vascular bed is dilated, which decreases the pressure. This can not be detected today by an IMD that does not have a cardiomechanic sensor according to the embodiments. Thus, embodiments can detect both types of vasovagal syncope by measuring MAP or diastolic blood pressure. By increasing the heart rate by pacing syncope episodes can be avoided.

Thus, the embodiments as disclosed herein can be used to control the delivered therapy by the IMD and diagnosis, for instance detection of syncope, arrhythmia detection and discrimination, drug titration of beta-blockers, detection of physical and emotional stress, long term pressure variability statistics can be collected, detection of pressure alternans, such as alternans of 2:1 patterns, can be an early marker of future severe arrhythmia events.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1.   An implantable medical device (100) comprising:

    a lead connecting arrangement (110) configured to be connected to a cardiac lead (210, 220) to be implanted in or in connection with a ventricle (12, 14) of a heart (10) of a patient (1) and comprising a cardiomechanic sensor (250) configured to generate a deformation signal representative of the myocardial deformation;
    a derivative calculator (131) configured to generate a deformation rate signal representative of rate of said myocardial deformation by calculating the derivative of said deformation signal with respect to time;
    a signal filter unit (150); and
    a pressure calculator (133), **characterized in that** said signal filter unit (150) is configured to filter said deformation rate signal from said derivative calculator (131) to get a filtered deformation rate signal, said implantable medical device (100) further comprises a peak identifier (132) configured to identify respective maximum deformation rate values in said filtered deformation rate signal for multiple cardiac cycles, wherein said pressure calculator (133) is configured to calculate a value representative of systemic blood pressure of said patient (1) based on a combination of said respective maximum deformation rate values identified by said peak identifier (132).

2.   The implantable medical device according to claim 1, **characterized in that** said signal filter unit (150) comprises a high pass filter (151) configured to high pass filter said deformation rate signal to remove or suppress any DC component from said deformation rate signal.

3.   The implantable medical device according to claim 1 or 2, **characterized in that** said signal filter unit (150) comprises a low pass filter (153) configured to low pass filter said deformation rate signal to remove or suppress noise from said deformation rate signal.

4.   The implantable medical device according to any of the claims 1 to 3, **characterized in that** said lead connecting arrangement (110) is electrically connectable-to at least one electrode (212, 214, 222, 224, 232, 234) provided on said cardiac lead (210, 220) or another cardiac lead (230) connectable to said lead connecting arrangement (110), said implantable medical device (100) further comprising:

    a data acquisition unit (160) connected to said lead connecting arrangement (110) and configured to generate a signal representative of electric activity of at least a portion of said heart (10); and
    a cardiac cycle identifier (134) configured to identify, for each cardiac cycle of said multiple cardiac cycles and based on said signal representative of said electric activity, a start point and an end point of said cardiac cycle in said filtered deformation rate signal, wherein said peak identifier (132) is configured to identify, for each cardiac cycle of said multiple cardiac cycles, a maximum deformation rate value in said filtered deformation rate signal from said start point to said end point of said cardiac cycle.

5.   The implantable medical device according to any of the claims 1 to 4, **characterized in that** said pressure calculator (133) is configured to calculate a signal representative of said systemic blood pressure as a moving average of multiple consecutive maximum deformation rate values identified by said peak identifier in said filtered deformation rate signal.

6.   The implantable medical device according to any of the claims 1 to 5, **characterized by** an absolute pressure calculator (135) configured to calculate an absolute value representative of an absolute systemic blood pressure of said patient (10) based on said value calculated by said pressure calculator (133) and a scaling factor and an offset factor.

7.   The implantable medical device according to claim 6, **characterized in that** said absolute pressure calculator (135) is configured to calculate an absolute mean arterial pressure, MAP, value representative of an absolute MAP of said patient (10) based on said value calculated by said pressure calculator (133) and a MAP scaling factor and a MAP offset factor.

8.   The implantable medical device according to claim 6 or 7, **characterized in that** said absolute pressure calculator (135) is configured to calculate an absolute pulse pressure, PP, value representative of an absolute PP of said patient (10) based on said value calculated by said pressure calculator (133) and a PP scaling factor and a PP offset factor.

9. The implantable medical device according to any of the claims 1 to 8, **characterized in that** said lead connecting arrangement (110) is configured to be connected to a cardiac lead (210) to be implanted in a coronary vein (11) of said heart (10) and comprising said cardiomechanic sensor (250).

10. The implantable medical device according to any of the claims 1 to 9, **characterized in that** said cardiomechanic sensor (250) is a cardiomechanic electric sensor, CMES (250).

11. The implantable medical device according to claim 10, **characterized in that** said CMES (250) comprises a piezoelectric element (251) having a first conductor (252) connected to an outer surface of said piezoelectric element (251) and a second conductor (253) connected to an opposite, inner surface of said piezoelectric element (251), said first conductor (252) and said second conductor (253) are configured to be electrically connected using respective electrical connections (254, 255) to said lead connecting arrangement (110).

**Patentansprüche**

1. Implantierbares Medizinprodukt (100), umfassend:

eine Leitungsanschlussanordnung (110), die so ausgelegt ist, dass sie an eine Herzleitung (210, 220) angeschlossen wird, die in eine oder in Verbindung mit einer Kammer (12, 14) eines Herzens (10) eines Patienten (1) implantiert wird und einen kardiomechanischen Sensor (250) umfasst, der so ausgelegt ist, dass er ein Verformungssignal erzeugt, das die Myokardverformung darstellt;
einen Ableitungsrechner (131), der so ausgelegt ist, dass er ein Verformungsgeschwindigkeitssignal, das die Geschwindigkeit der Myokardverformung darstellt, durch Berechnen der Ableitung des Verformungssignals nach der Zeit erzeugt;
eine Signalfiltereinheit (150); und
einen Druckrechner (133), **dadurch gekennzeichnet, dass** die Signalfiltereinheit (150) so ausgelegt ist, dass sie das Verformungsgeschwindigkeitssignal von dem Ableitungsrechner (131) filtert, damit sie ein gefiltertes Verformungsgeschwindigkeitssignal erhält, das implantierbare Medizinprodukt (100) ferner eine Spitzenerkennungseinheit (132) umfasst, die so ausgelegt ist, dass sie jeweilige Maximalwerte der Verformungsgeschwindigkeit in dem gefilterten Verformungsgeschwindigkeitssignal für mehrere Herzzyklen erkennt, wobei der Druckrechner (133) so ausgelegt ist, dass er einen Wert, der den systemischen Blutdruck des Patienten (1) darstellt, auf der Grundlage einer Kombination der jeweiligen Maximalwerte der Verformungsgeschwindigkeit berechnet, die von der Spitzenerkennungseinheit (132) erkannt werden.

2. Implantierbares Medizinprodukt nach Anspruch 1, **dadurch gekennzeichnet, dass** die Signalfiltereinheit (150) ein Hochpassfilter (151) umfasst, das so ausgelegt ist, dass das Verformungsgeschwindigkeitssignal hochpassgefiltert wird, damit Gleichstromanteile aus dem Verformungsgeschwindigkeitssignal entfernt oder unterdrückt werden.

3. Implantierbares Medizinprodukt nach Anspruch 1 oder 2, **dadurch gekennzeichnet, dass** die Signalfiltereinheit (150) ein Tiefpassfilter (153) umfasst, das so ausgelegt ist, dass das Verformungsgeschwindigkeitssignal tiefpassgefiltert wird, damit Rauschen aus dem Verformungsgeschwindigkeitssignal entfernt oder unterdrückt wird.

4. Implantierbares Medizinprodukt nach einem der Ansprüche 1 bis 3, **dadurch gekennzeichnet, dass** die Leitungsanschlussanordnung (110) an mindestens eine Elektrode (212, 214, 222, 224, 232, 234) elektrisch anschließbar ist, die an der Herzleitung (210, 220) oder einer weiteren Herzleitung (230) vorgesehen ist, die an die Leitungsanschlussanordnung (110) anschließbar ist, wobei das implantierbare Medizinprodukt (100) ferner Folgendes umfasst:

eine Datenerfassungseinheit (160), die mit der Leitungsanschlussanordnung (110) verbunden und so ausgelegt ist, dass sie ein Signal erzeugt, das eine elektrische Aktivität von zumindest einem Abschnitt des Herzens (10) darstellt; und
eine Herzzyklus-Erkennungseinheit (134), die so ausgelegt ist, dass sie, für jeden Herzzyklus der mehreren Herzzyklen und auf der Grundlage des Signals, das die elektrische Aktivität darstellt, einen Anfangspunkt und einen Endpunkt des Herzzyklus in dem gefilterten Verformungsgeschwindigkeitssignal erkennt, wobei die Spitzenerkennungseinheit (132) so ausgelegt ist, dass sie, für jeden Herzzyklus der mehreren Herzzyklen, einen Maximalwert der Verformungsgeschwindigkeit in dem gefilterten Verformungsgeschwindigkeitssignal vom Anfangspunkt bis zum Endpunkt des Herzzyklus erkennt.

**5.** Implantierbares Medizinprodukt nach einem der Ansprüche 1 bis 4, **dadurch gekennzeichnet, dass** der Druckrechner (133) so ausgelegt ist, dass er ein Signal, das den systemischen Blutdruck darstellt, als gleitenden Mittelwert mehrerer aufeinanderfolgender Maximalwerte der Verformungsgeschwindigkeit, die von der Spitzenerkennungseinheit in dem gefilterten Verformungsgeschwindigkeitssignal erkannt werden, berechnet.

**6.** Implantierbares Medizinprodukt nach einem der Ansprüche 1 bis 5, **gekennzeichnet durch** einen Absolutdruckrechner (135), der so ausgelegt ist, dass er einen Absolutwert, der einen absoluten systemischen Blutdruck des Patienten (10) darstellt, auf der Grundlage des Werts, der von dem Druckrechner (133) berechnet wird, und eines Skalierungsfaktors und eines Offset-Faktors berechnet.

**7.** Implantierbares Medizinprodukt nach Anspruch 6, **dadurch gekennzeichnet, dass** der Absolutdruckrechner (135) so ausgelegt ist, dass er einen absoluten mittleren arteriellen Druckwert, MAD, der einen absoluten MAD des Patienten (10) darstellt, auf der Grundlage des Werts, der von dem Druckrechner (133) berechnet wird, und eines MAD-Skalierungsfaktors und eines MAD-Offset-Faktors berechnet.

**8.** Implantierbares Medizinprodukt nach Anspruch 6 oder 7, **dadurch gekennzeichnet, dass** der Absolutdruckrechner (135) so ausgelegt ist, dass er einen absoluten Pulsdruckwert, PD, der einen absoluten PD des Patienten (10) darstellt, auf der Grundlage des Werts, der von dem Druckrechner (133) berechnet wird, und eines PD-Skalierungsfaktors und eines PD-Offset-Faktors berechnet.

**9.** Implantierbares Medizinprodukt nach einem der Ansprüche 1 bis 8, **dadurch gekennzeichnet, dass** die Leitungsanschlussanordnung (110) so ausgelegt ist, dass sie an eine Herzleitung (210) angeschlossen wird, die in eine Koronarvene (11) des Herzens (10) implantiert werden soll und den kardiomechanischen Sensor (250) umfasst.

**10.** Implantierbares Medizinprodukt nach einem der Ansprüche 1 bis 9, **dadurch gekennzeichnet, dass** es sich bei dem kardiomechanischen Sensor (250) um einen kardiomechanischen elektrischen Sensor, KMES (250), handelt.

**11.** Implantierbares Medizinprodukt nach Anspruch 10, **dadurch gekennzeichnet, dass** der KMES (250) ein Piezoelement (251) mit einem ersten Leiter (252), der an eine Außenfläche des Piezoelements (251) angeschlossen ist, und einem zweiten Leiter (253), der an eine gegenüberliegende Innenfläche des Piezoelements (251) angeschlossen ist, umfasst, der erste Leiter (252) und der zweite Leiter (253) so ausgelegt sind, dass sie unter Verwendung jeweiliger elektrischer Anschlüsse (254, 255) an die Leitungsanschlussanordnung (110) elektrisch angeschlossen werden.

**Revendications**

**1.** Dispositif médical implantable (100) comprenant :

un agencement de connexion de dérivation (110) configuré pour être connecté à une dérivation cardiaque (210, 220) destinée à être implantée dans ou en connexion avec un ventricule (12, 14) d'un coeur (10) d'un patient (1) et comprenant un capteur cardiomécanique (250) configuré pour générer un signal de déformation représentatif de la déformation du myocarde ;
un calculateur de dérivée (131) configuré pour générer un signal de vitesse de déformation représentatif de la vitesse de ladite déformation du myocarde en calculant la dérivée dudit signal de déformation en fonction du temps ;
une unité de filtre de signal (150) ; et
un calculateur de pression (133), **caractérisé en ce que** ledit filtre de signal (150) est configuré pour filtrer ledit signal de vitesse de déformation dudit calculateur de dérivée (131) pour obtenir un signal de vitesse de déformation filtré, ledit dispositif médical implantable (100) comprend en outre un identificateur de pic (132) configuré pour identifier des valeurs de vitesse de déformation maximales respectives dans ledit signal de vitesse de déformation filtré pour plusieurs cycles cardiaques ; dans lequel ledit calculateur de pression (133) est configuré pour calculer une valeur représentative de la pression artérielle systémique dudit patient (1) sur la base d'une combinaison desdites valeurs de vitesse de déformation maximales respectives identifiées par ledit identificateur de pic (132).

**2.** Dispositif médical implantable selon la revendication 1, **caractérisé en ce que** ladite unité de filtre de signal (150) comprend un filtre passe-haut (151) configuré pour filtrer en passe-haut ledit signal de vitesse de déformation pour retirer ou supprimer toute composante CC dudit signal de vitesse de déformation.

**3.** Dispositif médical implantable selon la revendication 1 ou 2, **caractérisé en ce que** ladite unité de filtre de signal (150) comprend un filtre passe-bas (153) configuré pour filtrer en passe-bas ledit signal de vitesse de déformation pour retirer ou supprimer du bruit dudit signal de vitesse de déformation.

**4.** Dispositif médical implantable selon l'une quelconque des revendications 1 à 3, **caractérisé en ce que** ledit agencement de connexion de dérivation (110) peut être connecté électriquement à au moins une électrode (212, 214, 222, 224, 232, 234) se trouvant sur ladite dérivation cardiaque (210, 220) ou une autre dérivation cardiaque (230) pouvant être connectée audit agencement de connexion de dérivation (110), ledit dispositif médical implantable (100) comprenant en outre :

une unité d'acquisition de données (160) connectée audit agencement de connexion de dérivation (110) et configurée pour générer un signal représentatif d'une activité électrique d'au moins une partie dudit coeur (10) ; et un identificateur de cycle cardiaque (134) configuré pour identifier, pour chaque cycle cardiaque desdits plusieurs cycles cardiaques et sur la base dudit signal représentatif de ladite activité électrique, un point de départ et un point de fin dudit cycle cardiaque dans ledit signal de vitesse de déformation filtré, dans lequel ledit identificateur de pic (132) est configuré pour identifier, pour chaque cycle cardiaque desdits plusieurs cycles cardiaques, une valeur de vitesse de déformation maximale dans ledit signal de vitesse de déformation filtré dudit point de départ audit point de fin dudit cycle cardiaque.

**5.** Dispositif médical implantable selon l'une quelconque des revendications 1 à 4, **caractérisé en ce que** ledit calculateur de pression (133) est configuré pour calculer un signal représentatif de ladite pression artérielle systémique en tant que moyenne mobile de plusieurs valeurs de vitesse de déformation maximales consécutives identifiées par ledit identificateur de pic dans ledit signal de vitesse de déformation filtré.

**6.** Dispositif médical implantable selon l'une quelconque des revendications 1 à 5, **caractérisé par** un calculateur de pression absolue (135) configuré pour calculer une valeur absolue représentative d'une pression artérielle systémique absolue dudit patient (10) sur la base de ladite valeur calculée par ledit calculateur de pression (133) et d'un facteur d'échelle et d'un facteur de décalage.

**7.** Dispositif médical implantable selon la revendication 6, **caractérisé en ce que** ledit calculateur de pression absolue (135) est configuré pour calculer une valeur de pression artérielle moyenne, PAM, absolue représentative d'une PAM absolue dudit patient (10) sur la base de ladite valeur calculée par ledit calculateur de pression (133) et d'un facteur d'échelle de PAM et d'un facteur de décalage de PAM.

**8.** Dispositif médical implantable selon la revendication 6 ou 7, **caractérisé en ce que** ledit calculateur de pression absolue (135) est configuré pour calculer une valeur de pression pulsée, PP, absolue représentative d'une PP absolue dudit patient (10) sur la base de ladite valeur calculée par ledit calculateur de pression (133) et d'un facteur d'échelle de PP et d'un facteur de décalage de PP.

**9.** Dispositif médical implantable selon l'une quelconque des revendications 1 à 8, **caractérisé en ce que** ledit agencement de connexion de dérivation (110) est configuré pour être connecté à une dérivation cardiaque (210) destinée à être implantée dans une veine coronaire (11) dudit coeur (10) et comprenant ledit capteur cardiomécanique (250).

**10.** Dispositif médical implantable selon l'une quelconque des revendications 1 à 9, **caractérisé en ce que** ledit capteur cardiomécanique (250) est un capteur électrique cardiomécanique, CECM (250).

**11.** Dispositif médical implantable selon la revendication 10, **caractérisé en ce que** ledit CECM (250) comprend un élément piézoélectrique (251) comportant un premier conducteur (252) connecté à une surface extérieure dudit élément piézoélectrique (251) et un second conducteur (253) connecté à une surface intérieure opposée dudit élément piézoélectrique (251), ledit premier conducteur (252) et ledit second conducteur (253) sont configurés pour être connectés électriquement en utilisant des connexions électriques (254, 255) respectives audit agencement de connexion de dérivation (110).

Fig. 1

Fig. 3A

Fig. 2

Fig. 3B

Fig. 4

Fig. 5A   Fig. 5B   Fig. 5C   Fig. 5D

Fig. 6

Fig. 7

Fig. 8

Fig. 9

Fig. 10

Fig. 11

START

S1 — GENERATE DEFORMATION RATE SIGNAL

S2 — FILTER DEFORMATION RATE SIGNAL

S3 — IDENTIFY MAX DEFORMATION RATE VALUES

S4 — CALCULATE PRESSURE VALUE

STOP

Fig. 12

FROM STEP S1

S10 — HIGH PASS FILTER DEFORMATION RATE SIGNAL

S11 — LOW PASS FILTER DEFORMATION RATE SIGNAL

TO STEP S3

Fig. 13

FROM STEP S2

S20 — SENSE ELECTRIC ACTIVITY OF HEART

S21 — GENERATE IEGM SIGNAL

S22 — IDENTIFY CYCLE START AND END

TO STEP S3

Fig. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0228478 A
- US 5129394 A
- US 6666826 B
- US 6915162 B
- US 4566456 A
- US 6526984 B
- US 20090312814 A